# EUROPEAN PATENT SPECIFICATION

(11) **EP 2 079 408 B1**
(45) Date of publication and mention of the grant of the patent: **21.12.2011**
(21) Application number: 07805571.2
(22) Date of filing: 10.09.2007
(51) Int. Cl.: A61F 5/00

(54) **INFLATABLE SPLINT**
AUFBLASBARE SCHIENE
ATTELLE GONFLABLE

(30) Priority: 14.09.2006 US 844383 P
(43) Date of publication of application: 22.07.2009
(73) Proprietor: Ortho-Flex Ltd., 34369 Haifa (IL)
(72) Inventor: REIS, Daniel, 34656 Haifa (IL); ZUCKER, Dalia, 34890 Haifa (IL); BIN NUN, Asher, 21721 Carmiel (IL)
(74) Representative: Becker Kurig Straus
(86) International application number: PCT/IL2007/001111
(87) International publication number: WO 2008/032309

(56) References cited:
- WO-A-2005/084136
- US-A- 5 957 872
- US-A1- 2003 191 420
- US-A1- 2006 189 907

## Description

### FIELD OF THE INVENTION

The present invention relates to an inflatable splint which may be used, for example, in emergency situations, before and after surgical operations on the limbs, fractures, intensive care, and heel spurs.

### BACKGROUND OF THE INVENTION

A splint is a medical device for the immobilization of limbs. It can be used by the emergency medical services or by volunteer first responders, to immobilize a fractured limb before the transportation; it is then a temporary immobilization. Splints can also be used by nurses and physicians in an emergency room, operation room, intensive care unit, or by a physiotherapist, via a physician's order, to support or immobilize an articulation (e.g. the ankle).

As many injuries and fractures to legs occur in locations that are, distant from immediate, competent medical attention, and transportation of the injured person may be hazardous and painful resulting in further injury to the fractured leg, the splint should meet such requirements as size, ease of application and protection of the injured limb, to be capable of being folded into a compact small size that can fit into a first-aid kit to be easily carried in an ambulance or the like, and to be capable of being properly applied by a person other than a medical doctor and without further injury or damage to the broken leg. Various such splint devices have been developed. Examples of inflatable splint devices are disclosed in U.S. Patent No. 5,957,872, and in PCT Application WO 2005/084136.

US Patent Application 2003/0191420 discloses a therapeutic limb covering and an associated method of treating chronic swelling of a limb. The limb covering is uses hydrostatic pressure provided by liquid contained within the covering to apply pressure to the limb. The limb covering comprises a substantially non-distensible flexible outer layer, a distensible flexible inner layer joined together and a liquid tight bladder therebetween. The covering may be adapted for releasable securement about a limb such as an arm or a lower leg and foot of a patient. After placement on the limb, when the bladder is filled with a liquid, such as water, it expands to contact and apply pressure to the limb.

US Patent 5,957,872 discloses a protective and pressure normalizing device for a body extremity, that has a topside, an underside and two other sides. The device has an inflatable member, a non-inflatable member, a heel aperture, releasable securing means and a nozzle. The inflatable member has a lower portion, an intermediate portion, and an upper portion. Each portion contacts at least a portion of the other sides of the respective lower, intermediate, and upper region of the extremity. The heel aperture exposes the underside of the intermediate region. The non-inflatable member contacts at least a portion of the underside of the lower region. The inflatable member and heel aperture surround at least a portion of the non-inflatable member. The releasable securing means secures the device at least about the lower portion. The device engages and partially encloses in a cradling fashion the extremity. Thereby, the inflating member applies a uniform interface pressure where it is applied to the extremity.

US-A-2006-189 907 discloses a boot with inflatable walls for supporting a patients leg. The foot portion has a stiff support plate with a soft pad disposed on said plate. No face of the lower leg or foot is exposed when the boot is fitted over this leg part.

### SUMMARY OF THE INVENTION

The present invention is concerned with a new inflatable splint as defined in claim 1 . The splint of the present invention is useable for fixation or support of a leg part. The leg part includes at least the lower part of the leg ("lower leg") including at least the foot, the ankle and the calf, or the whole leg up to above the knee.

The inflatable splint of the invention includes either one continuous air pocket or a plurality of air pockets (tubes) that can be inflated, whereupon the splint assumes a cradle form for the lower limb in which it is relatively stiff, and can optionally assume variable degrees of stiffness to be used as a leg fixation or support. The stiffness of the brace is varied according to the pressure of the inflation. When folded before inflation, the splint is a small flat package.

In the following, some embodiments of the invention are described. These embodiments may be applied separately or each embodiment may be applied in combination with one or more of the other defined embodiments.

In accordance with one embodiment of the invention the splint is configured to have a collapsed or folded state and an open state and once opened the splint assumes the general form of the leg part. This is of advantage as once opened the splint is an immediate configuration for mounting on the leg. The splint may be inflated before or after mounting. Also, at times the splint may be partially inflated before mounting and then inflated to a final desired degree, e.g. based on medical considerations, after mounting.

In accordance with this embodiment there is thus provided an inflatable splint comprising integral foot-accommodating portion, a calf-accommodating portion and an intermediate, ankle-accommodating portion; the splint being configured to be fitted over a leg part comprising a foot, ankle and calf such that front faces of the leg part are exposed; the splint having a collapsed state and having an open state in which it can be mounted on the leg part; when opened the splint assumes the general form of the leg part.

As indicated above, the splint assumes the general form of the leg part (at least lower leg) once opened, both in its inflated and non-inflated state.

In accordance with the invention the splint is provided with a rigid foot floor-support panel and a foot cushion disposed above the panel. This imparts rigid support for the foot and more evenly distributed pressure on the foot, on the other hand.

In accordance with this embodiment there is thus provided an inflatable splint comprising integral foot-accommodating portion, a calf-accommodating portion and an intermediate, ankle-accommodating portion, the splint being configured to be fitted over a leg part comprising a foot, ankle and calf such that front faces of the leg are exposed; said foot-accommodating portion has a rigid floor-support panel and a foot cushion, typically although not exclusively, inflatable, disposed above said panel.

In accordance with another embodiment of the invention the splint is provided with adjustable fasteners which have one of their ends connected to the side of one of the splint's portions in a releasable arrangement. This permits rapid mounting of the splint over a patient's leg part.

In accordance with this embodiment there is thus provided an inflatable splint comprising integral foot-accommodating portion, a calf-accommodating portion and an intermediate, ankle-accommodating portion; the splint being configured to be fitted over a leg part comprising a foot, ankle and calf such that front faces of the leg part are exposed; the splint portions having side surfaces that in use are disposed over sides of the leg part with edges that leave the front faces exposed; said side surfaces being provided with one or more first adjustable fasteners that link opposite side surfaces to one another; one end of the first fasteners being linked to an anchor member that is connected in a releasable arrangement to one of the side surfaces.

The releasable arrangement may include a zipper but may also include other types of arrangement such as that which includes a hook-and-pile couple (e.g. one known as *"Velcro*®")or any kind of quick release buckle, one of which is on a member connected to said end of the fastener arrangement and the other to said one of the sides surfaces.

The first fasteners, in the above embodiment, are typically disposed on side surfaces of the calf-accommodating portion. In some embodiments of the invention, one or more second fasteners are provided that link opposite side surfaces of the foot-accommodation portion.

In accordance with another embodiment of the invention a novel outrigger stabilizer member is provided that can be attached to the splint in a detachable manner for inhibiting axial rotation of a fixed patient's leg. The outrigger attachment controls the limb axis rotation.

In accordance with this embodiment there is thus provided an inflatable splint comprising integral foot-accommodating portion, a calf-accommodating portion and an intermediate, ankle-accommodating portion; the splint being configured to be fitted over a leg part comprising a foot, ankle and calf such that front faces of the leg part are exposed; the splint having a collapsed state and having an open state in which it can be mounted on the leg part; the splint being provided with an outrigger stabilizer member configured for attachment in a detachable manner to a bottom face of the foot-accommodating portion and having an edge that once connected is positioned at the rear of the foot-accommodating portion, and projects laterally from the splint.

A typical detachable arrangement is through a hook-and-pile couple arrangement. Typically, said outrigger stabilizer member has an attachment surface that extends lengthwise over a substantial part of the bottom face. For example, the attachment surface of the outrigger stabilizer member may be fitted with one member of the hook-and-pile couple, and the other member of said couple being disposed on said bottom face. According to some embodiments of the invention, the outrigger stabilizer member comprises a tongue that extends rearwards from said edge and is configured for attachment to an attachment surface in the rear face of the ankle-accommodating portion of the splint.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Figs. 1-3** shows a splint in accordance with an embodiment of the invention in which Fig. 1 shows the splint in a closed state, Fig. 2 shows the splint, in a non-inflated state, and Fig. 3 shows the splint in an inflated state.
**Fig. 4A** is a partial longitudinal cross-section through the lower parts of the splint according to an embodiment of the invention, open and in a non-inflated state.
**Fig. 4B** is a partial cross-section through the lower part of a splint fitted with a heel raise pad.
**Fig. 4C** illustrates an inflatable splint according to an embodiment of the invention having a heel raise pad and pad extensions along the sides of the inner surface of the foot-accommodating portion.
**Fig. 5** is a partial transverse cross-section through the lower part of the splint of **Fig. 4A**, in an inflated state.
**Fig. 6** is a splint according to an embodiment of the invention in which the bottom of the foot-accommodating portion has a pocket for holding a folded outrigger stabilizer member shown removed from the pocket.
**Fig. 7** shows the outrigger in an open, unfolded state.
**Fig. 8** is a side perspective view of a splint, in an inflatable state, fitted with an outrigger member.
**Fig. 9** shows a view from the direction of arrow XI in Fig. 8.

### DETAILED DESCRIPTION OF AN EXEMPLARY EMBODIMENT

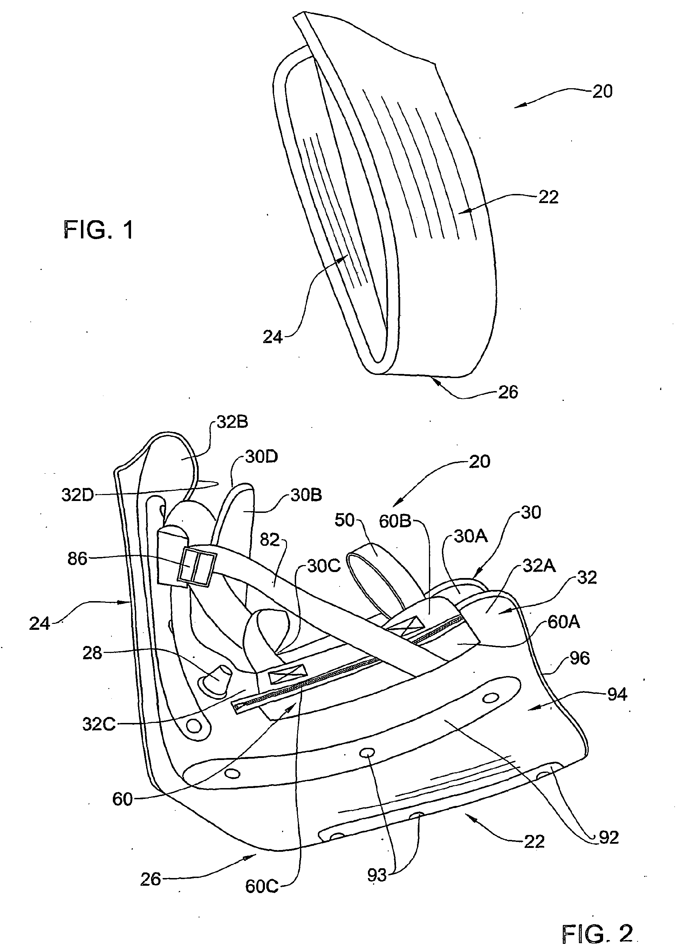

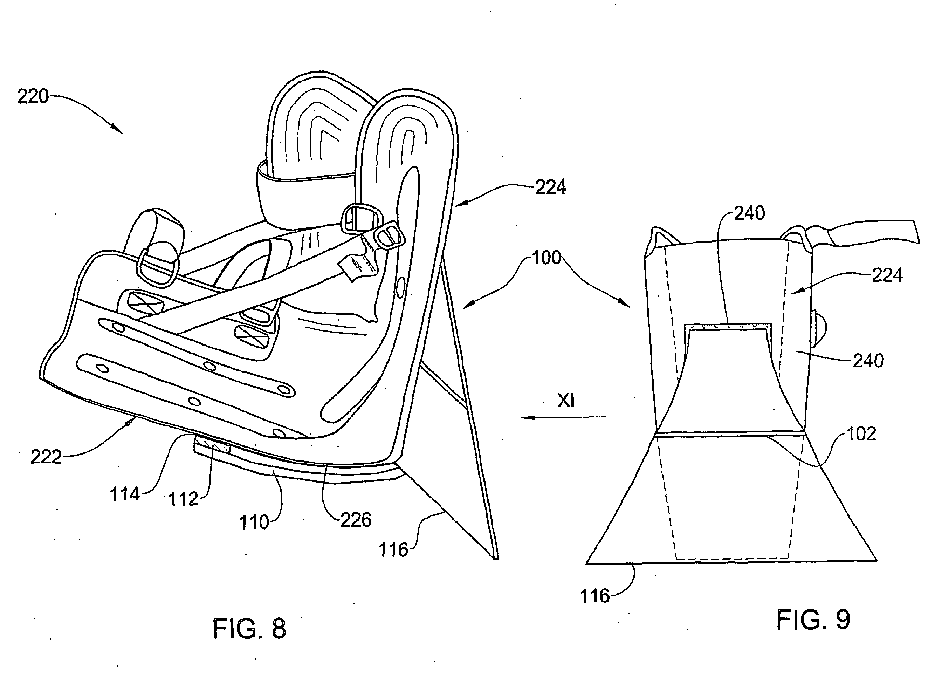

Exemplary embodiments of the invention are shown in and described with reference to **Figs. 1-9**. As will be appreciated, these embodiments while being shown for purpose of illustration of the various features of the invention as claimed herein, is non-limiting and various modifications of this embodiment, as will be clear to a person versed in the art, are possible.

Shown in **Figs. 1-3** is a splint generally designated as **20** having a body including a calf-accommodating portion **22**, a foot-accommodating portion **24** integrally formed and linked together through an intermediate ankle-accommodating portion **26.** In the closed state shown in **Fig. 1**, the splint is folded about the heel part of the ankle-accommodating portion **26** to assume a relatively compact configuration of a small flat package in which it can be stored until use.

As can be seen in **Fig. 2**, the splint includes a filling nozzle to permit introduction of inflation fluid, which may be a liquid or a gas, typically air. The advantage of using a gas is that even while fully inflated, inflated pockets in the splint, to be described below, will have some residual flexibility and a cushion effect. A liquid as an inflation fluid may be useful, for example, for applications in which a cooling effect is desired. Furthermore, the filling with a liquid will render the inflated splint to be heavier than the case of a gas, such as air, such weight being of advantage for one application while light weight of a gas-inflated splint being advantageous for others.

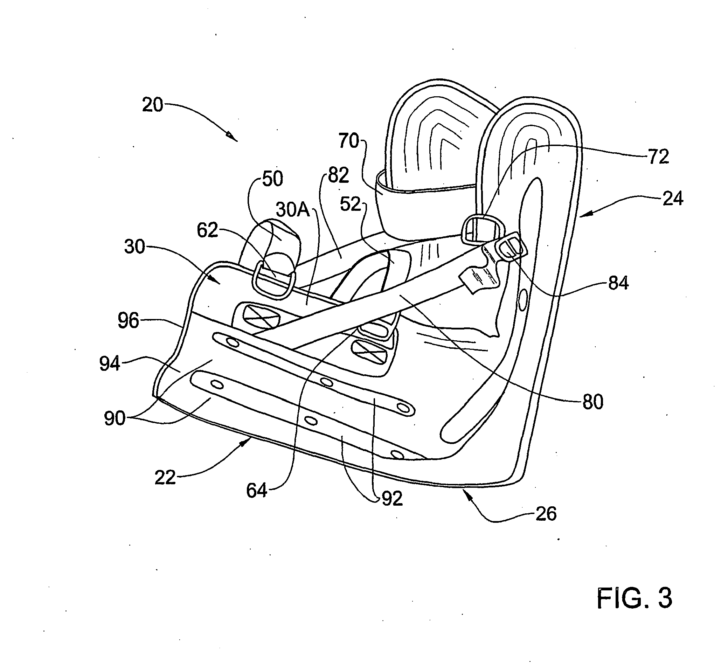

In **Fig**. **2** the splint is shown in an open, yet non-inflated state. As can be seen in this figure, once opened the splint has already a general boot-like form in which in can be fitted over the leg part (lower leg in the present example). In this form the calf-accommodating portion **22** is essentially normal to the foot-accommodating portion **24.** Upon inflation with a fluid, typically, although not exclusively, air, as shown in **Fig. 3****,** the splint becomes rigid. In an inflated state the fluid containing pockets of the splint expand outwards and also towards the splint interior whereby they create a supporting cradle for the subject's leg.

As can be seen in **Figs**. **2** **and** **3**, the splint is formed with side surfaces that include a right side surface **30** and a left side surface **32**, each of which being provided with respectable first side surfaces **30A** and **32A**, which can fit over the sides of a subject's calf, second respective side surfaces **30B** and **32B**, which are intended to fit over sides of a foot and intermediate respective side surfaces **30C** and **32C**, fitting over sides of the ankle. The side surfaces have respective edges **30D** and **32D** that define therebetween an open space for exposure of front faces of the leg part.

The splint shown in **Figs**. **1-3** is provided, in this specific embodiment, with a number of inflatable tubes, in fluid communication with one another, such as that disclosed in WO 2005/084136. Thus, all air pockets can be inflated through fluid ingress through nozzle **28**. As will be appreciated the position of the nozzle **28** is but an example and the nozzle may be positioned in any suitable place in the splint.

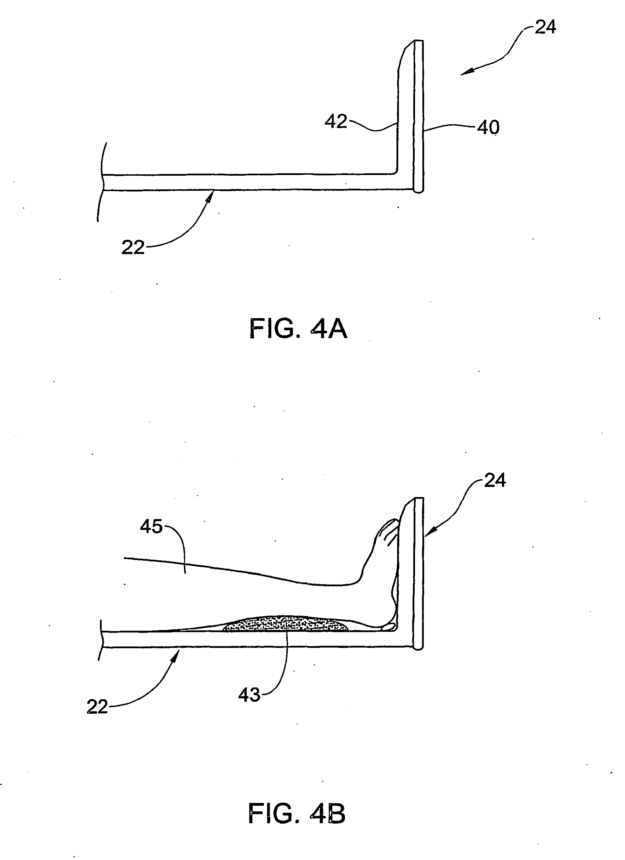

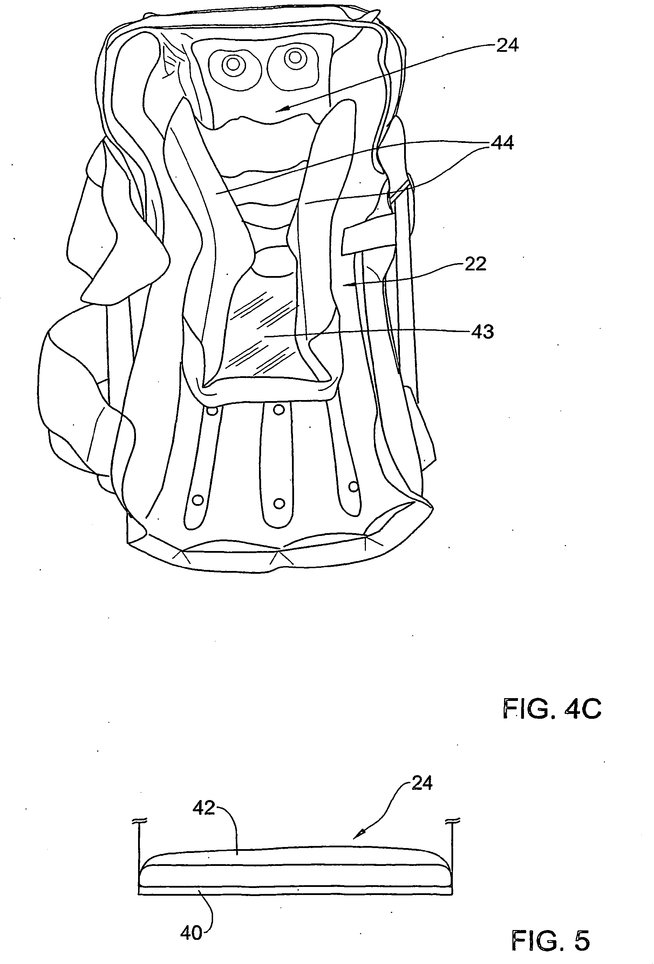

Reference is now being made to **Figs**. **4A** **and** **5**, where **Fig**. **4A** is a partially longitudinal cross-section of the splint in a non-inflated state and **Fig**. **5** is a transverse cross-section through the sole portion (foot-accommodating portion) **24** in an inflated state of the same splint. As can be seen in Figs. 4A and 5, the foot-accommodating portion **24** is provided with a rigid floor support panel **40** overlaid with a foot cushion **42** which in accordance with one embodiment of the invention is inflatable. However, it should be noted that the foot cushion may be formed also, for example, from a flexible, non-inflatable matrix, typically covered or completely enclosed within a sheet which may be made of a cloth, liquid impermeable polymer, etc.

As can be seen in **Figs**. **4B** **and** **4C**, the splint may also be provided with an optional heel raise pad **43**, placed immediately behind and bordering on the special heel depression in the nylon of the heel portion, and possibly also pad extensions **44** along the sides of the inner surface of the foot-accommodating portion **24** which protect the heel and the borders of the foot from pressure injury. The heel raise pad (e.g. with its extensions) may be inflatable or may be made from a soft flexible non-inflatable matrix. In Fig. 4B, pad **43** is shown as raising the heel part in lower leg **45**. In accordance with one embodiment the pad **43** is fixed within the splint. In accordance with another embodiment the pad is removable.

As can be seen in **Figs**. **2** **and** **3**, the first side surfaces **30A** and **32A** are connectable to one another through two length-adjustable straps **50** and **52**. It should be appreciated that the adjustable straps are one of many examples of fasteners that may be used for connecting the two side surfaces to one another, such as for example, strings, laces and others. Furthermore, as can be appreciated, the number of straps or other fasteners may be different than two; there may be one fastener according to some embodiment or a plurality, more than two fasteners according to others.

Fasteners **50** and **52** are fixed at one of their ends to an anchor member **60**, which is secured at side surface **32A** and connected at their other end to side surface **30A** through buckles **62** and **64**. In use, the straps **50** fit over the front face of a subject's calf and ankle and serve to secure the splint in position.

Anchor member **60** is a two part member consisting of parts **60A** and **60B** releasably connected to one another through zipper **60C**. Through opening of zipper **60C** the part **60B** with the associated straps can be released to permit easy fitting of the splint onto a subject's leg and then after fitting rapid securing of the straps over the subject's front leg surfaces. The rapid application arrangement, such as one comprising a zipper **60C** is especially important in long leg versions of the splint. The straps **50** and **52** are connected and are length adjustable through respective buckles **62** and **64**. As will be appreciated a buckle arrangement is one of many length adjustable fastening arrangements known *per se.*

It should be noted that the use of a zipper is only one option for releasable anchor arrangement and other arrangements such as that which make use of a hook and pile couple, are possible.

Optionally, the splint is provided also with an adjustable fastening arrangement for linking second side surfaces **30B** and **32B**. As can be seen in **Fig. 3**, provided is a strap **70** that links the two side surfaces which is fixed to side surface **32B** and adjustably connected through buckle **72** to side surface **30B**.

Another feature according to a preferred embodiment of the invention is an adjustable strap that links the foot-accommodating portion and the calf-accommodating portion for dorsiflexing of the ankle joint. This can be seen in **Figs. 2** **and** **3** where straps **80** and **82** are provided which are fixed to the upper part of first side surfaces **30A** and **32A** and are adjustably fitted through respective buckles **84** and **86** to side surfaces **30B** and **32B**. Straps **80** and **82** are operative to maintain the ankle at neutral position, e.g. about 90 degrees, or when desired they can be tightened to dorsiflex the ankle creating tension in the heel cord and the plantar fascia of the sole of the foot.

Typically, the splint body is formed from two parallel polymeric sheets and the air pockets within the splint are formed through a series of spaced apart welds that define inflatable air pockets **90** between them, separated by non-inflatable portions, such as portions **92**. These non inflatable portions are perforated by a series of ventilation holes **93**. In accordance with an embodiment of the invention the welds are designed such as to provide a continuous inflatable portion **94** adjacent the splint's upper rim **96**. Preferably, as can be seen also in the embodiment herein, the continuous inflatable portion adjacent to the rim also extends along rims of the side portion **30**.

The body may be made of any suitable weldable material of a kind that can withstand the required pressure of the fluid within the inflatable portions without rupturing. In some embodiments of the invention the gas pressure is around 0.25 atmospheres and may reach as high as 1 atmosphere without leakage of gas. In accordance with some embodiments of the invention, the splint body is made of nylon coated with polyurethane.

The fasteners may be fixed to the splint body through a number of arrangements, among others depending on the material of the splint body and that of the fasteners. In the specific embodiment where the splint body is made from a polymeric material and the straps from a woven material, a typical, but not exclusive, manner of securing may be by sewing.

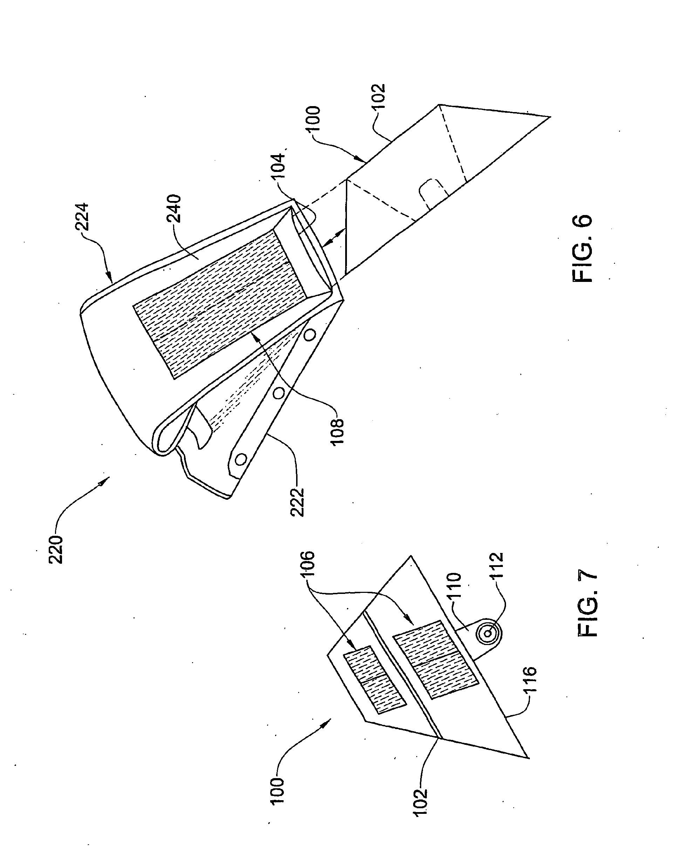

**Figs. 6-9** show an embodiment of the invention in which use is made of an outrigger stabilizer member **100.** In **Figs. 6**, **8 and 9** like reference numerals to those of **Figs. 1-3** are used, shifted by **200** to designate similar components. In the shown example, the outrigger member **100** is substantially rigid and is foldable about line **102** into a folded state as can be seen in **Fig**. **6**. In **Fig.** 6, splint **220** is shown in a partial folded state. Splint **220** seen in **Fig. 6** is provided with a pocket with a rear-end opening **104**, formed in the bottom of the foot-accommodating portion **222**. In a typical example, the pocket is a lengthwise space below the floor-support panel **240**.

The outrigger member **100**, has in the shown exemplary embodiment, and overall trapezoidal shape and is provided with and attachment device **106** which is designed for attachment to a corresponding attachment device **108** at the bottom of the leg accommodating portion **222** of the splint **220.** By a specific example, devices **106** and **108** are a hook-and-pile couple. The outrigger member **100** is preferably provided with a tongue **110**, extending from the rear edge **116**, having an attachment device **112**, for attaching to a corresponding attachment device **114**, in the rear face of the ankle-accommodating portion **226** shown in **Fig**. **8**.

**Figs**. **8 and 9** show the outrigger in its operative position being attached to the bottom face of the foot-accommodating portion with its rear edge **116**, being essentially aligned with the heel part of the ankle-accommodating portion **226** or slightly projecting in a rearward direction from the rear end of the bottom of the foot-accommodating portion (e.g. a 3-5 cm), and projecting laterally from the two sides of the splint. The tongue member **110** prevents the outrigger **100** from being tom off when the leg is moved, particularly when the leg is drawn up by flexing the thigh. The outrigger stabilizes the whole leg in neutral rotation and prevents the leg from rolling out or inwards. This is of particular importance in injuries of the thigh and hip.

The embodiment shown herein is designed for supporting the leg and the ankle. As will be appreciated, the invention also applies to inflatable splints for different purposes, such as one intended to support an entire leg and parts of or the whole of the upper limb.

## Claims

1. An inflatable splint (20, 220) configured to be fitted over a leg part comprising a foot, ankle and calf such that front faces of the leg part are exposed, the splint (20, 220) comprising integrally made portions comprising a foot-accommodating portion (24), a calf-accommodating portion (22) and an intermediate, ankle-accommodating portion (26), the splint (20, 220) having a collapsed state and having an open state in which it can be mounted on the leg part, when opened the splint (20, 220) assuming the general form of the leg part, the splint (20, 220) being **characterized in that** said foot-accommodating portion (24) has a rigid floor-support panel (40, 240) and a foot cushion (42) disposed above said panel.

2. An inflatable splint (20, 220) according to claim 1, configured such that it assumes the general form of the leg part in both inflated and non-inflated state.

3. An inflatable splint (20, 220) according to claim 1 or 2, wherein said foot cushion (42) disposed above said panel in the foot-accommodating portion (24) is inflatable.

4. An inflatable splint (20, 220) according to claim 3, comprising a pocket defined under the foot panel for accommodating at least one splint accessory.

5. An inflatable splint (20, 220) according to claim 4, wherein said accessory is an outrigger stabilizer member (100) for attachment to the splint (20, 220) to hinder axial rotation of the leg.

6. An inflatable splint (20, 220) according to any one of preceding claims, comprising a cushioned heel raise support pad (43).

7. An inflatable splint (20, 220) according to any one of preceding claims, wherein the splint (20, 220) portions have side surfaces (30, 32) that in use are disposed over sides of the leg part with edges (30D, 32D) that define therebetween the exposed front faces, said side surfaces (30, 32) being provided with one or more first adjustable fasteners that link opposite side surfaces to one another, one end of the first fasteners being linked to an anchor member (60) that is connected in a releasable arrangement to one of the side surfaces (30, 32).

8. An inflatable splint (20, 220) according to claim 7, **characterized by** at least one of the following:
(i) said releasable arrangement is a zipper (60C) or a hook-and-pile couple or a quick release buckle;
(ii) said first fasteners are disposed on side surfaces of the calf-accommodating portion (22); and
(iii) said first fasteners are disposed on side surfaces of the calf-accommodating portion (22), and one or more second fasteners are provided for linking opposite side surfaces of the foot-accommodating portion (24).

9. An inflatable splint (20, 220) according to any one of claims 5 to 8, wherein said outrigger stabilizer member (100) is configured to be attachable to the splint (20, 220) in a detachable manner, and configured for attachment to a bottom face of the foot-accommodating portion (24), the outrigger stabilizer (100) member having an edge that once connected is position at the rear of the foot-accommodating portion (24) and projects laterally from the splint (20, 220).

10. An inflatable splint (20, 220) according to claim 9, wherein the detachable attachment is through a hook-and-pile couple arrangement, said outrigger stabilizer member (100) having one of the following configurations:
(a) an attachment surface that extends lengthwise over a substantial part of said bottom face;
(b) a tongue (110) extending rearwards from said edge and being configured for attachment to an attachment surface in a rear face of the splint (20, 220); and
(c) said outrigger stabilizer (100) member being foldable.

11. An inflatable splint (20, 220) according to any one of preceding claims, comprising at least one of the following:
(i) adjustable straps (50, 52) between the foot-accommodating portion (24) and the ankle portion for dorsiflexing the ankle joint; and
(ii) inflatable and non-inflatable portions, said inflatable portions being designed to withstand pressures of up to 1 atmosphere without gas leakage or bursting.

12. An inflatable splint (20, 220) according to anyone of the preceding claims, comprising inflatable and non-inflatable portions, said inflatable portions being designed to withstand pressures of up to 1 atmosphere without gas leakage or bursting, wherein at least said inflatable portions are made of nylon coated with polyurethane.

13. An inflatable splint (20, 220) according to anyone of the preceding claims, comprising foot border protection pads.

14. An inflatable splint (20, 220) according to anyone of claims 6 to 13, wherein the heel raise support pad (43) has at least one of the following configurations:
is removable;
is inflatable;
is non- inflatable being made of a soft flexible matrix; and
is fixed within the splint (20, 220).

15. An inflatable splint (20, 220) according to any one of preceding claims, comprising inflatable and non-inflatable portions, said inflatable portions defining a substantially continuous inflatable belt adjacent an edge of the splint (20, 220).

## Patentansprüche

1. Aufblasbare Schiene (20, 220), die eingerichtet ist, um über ein Teil eines Beins, das einen Fuß, ein Fußgelenk und eine Wade umfasst, angebracht zu werden, sodass die Vorderseiten des Teils des Beins freiliegen,
wobei die Schiene (20, 220) einstückig ausgeführte Abschnitte umfasst, die einen Fuß-Aufnahmeabschnitt (24), einen Waden-Aufnahmeabschnitt (22) und einen zwischenliegenden Fußgelenk-Aufnahmeabschnitt (26) umfassen,
wobei die Schiene (20, 220) einen zusammengelegten Zustand und einen offenen Zustand aufweist, in dem sie an dem Teil des Beins angebracht werden kann,
wobei die Schiene (20, 220), wenn sie geöffnet ist, die allgemeine Form des Teils des Beins annimmt,
wobei die Schiene (20, 220) **dadurch gekennzeichnet ist, dass** der Fuß-Aufnahmeabschnitt (24) eine steife Boden-Stützplatte (40, 240) und eine Fußpolsterung (42) aufweist, die oberhalb der Platte angeordnet ist.

2. Aufblasbare Schiene (20, 220) gemäß Anspruch 1, die eingerichtet ist, sodass sie sowohl in einem aufgeblasenen Zustand als auch in einem nicht-aufgeblasenen Zustand die allgemeine Form des Teils des Beins annimmt.

3. Aufblasbare Schiene (20, 220) gemäß Anspruch 1 oder 2, wobei die über der Platte in dem Fuß-Aufnahmeabschnitt (24) angeordnete Fußpolsterung (42) aufblasbar ist.

4. Aufblasbare Schiene (20, 220) gemäß Anspruch 3, umfassend eine Tasche, die unter der Fußplatte definiert ist, um mindestens ein Schienenzubehörteil unterzubringen.

5. Aufblasbare Schiene (20, 220) gemäß Anspruch 4, wobei das Zubehörteil ein Ausleger-Stabilisierungselement (100) zum Anbauen an die Schiene (20, 220) umfasst, um eine axiale Drehung des Beins zu erschweren.

6. Aufblasbare Schiene (20, 220) gemäß einem der vorstehenden Ansprüche, umfassend eine gepolsterte Stützplatte (43) zum Anheben der Ferse.

7. Aufblasbare Schiene (20, 220) gemäß einem der vorstehenden Ansprüche, wobei die Abschnitte der Schiene (20, 220) Seitenflächen (30, 32) aufweisen, die im Gebrauch über Seiten des Teils des Beins angeordnet sind, mit Rändern (30D, 32D), die dazwischen die freiliegenden vorderen Flächen definieren, wobei die Seitenflächen (30, 32) mit einer oder mehreren ersten einstellbaren Befestigungen versehen sind, die gegenüberliegende Seitenflächen miteinander koppeln, wobei ein Ende der ersten Befestigungen mit einem Ankerelement (60) gekoppelt ist, das in lösbarer Ausgestaltung mit einem der Seitenflächen (30, 32) verbunden ist.

8. Aufblasbare Schiene (20, 220) gemäß Anspruch 7, **gekennzeichnet durch** mindestens eines der Folgenden:
(i) die lösbare Ausgestaltung ist ein Reißverschluss (60C) oder ein Klettverschluss oder ein Schnellverschluss;
(ii) die Befestigungen sind an Seitenflächen des Waden-Aufnahmeabschnitts (22) angeordnet; und
(iii) die Befestigungen sind auf Seitenflächen des Waden-Aufnahmeabschnitts (22) angeordnet und eine oder mehrere Befestigungen sind bereitgestellt, um gegenüberliegende Seitenflächen des Fuß-Aufnahmeabschnitts (24) zu koppeln.

9. Aufblasbare Schiene (20, 220) gemäß einem der vorstehenden Ansprüche 5 bis 8, wobei das Ausleger-Stabilisierungselement (100) eingerichtet ist, um abnehmbar an der Schiene (20, 220) angebaut zu werden, und eingerichtet ist, um an eine Bodenfläche des Fuß-Aufnahmeabschnitts (24) angebaut zu werden, wobei das Ausleger-Stabilisierungselement (100) einen Rand aufweist, der, wenn er einmal verbunden ist, hinten an dem Fuß-Aufnahmeabschnitt (24) positioniert ist und seitlich von der Schiene (20, 220) vorsteht.

10. Aufblasbare Schiene (20, 220) gemäß Anspruch 9, wobei die abnehmbare Anbringung durch ein Klettverschluss-Anordnung erreicht wird, wobei das Ausleger-Stabilisierungselement (100) eine der folgenden Konfigurationen aufweist:
(a) eine Anbringungsfläche, die sich in Längsrichtung über einen wesentlichen Teil der Bodenfläche erstreckt;
(b) eine Zunge (110), die sich von dem Rand nach hinten erstreckt und die eingerichtet ist, um an einer Anbaufläche in einer Hinterseite der Schiene (20, 220) angebaut zu werden; und
(c) das Ausleger-Stabilisierungselement (100) ist faltbar.

11. Aufblasbare Schiene (20, 220) gemäß einem der vorstehenden Ansprüche, umfassend mindestens eines der Folgenden:
(i) Einstellbare Bänder (50, 52) zwischen dem Fuß-Aufnahmeabschnitt (24) und dem Knöchelabschnitt, um das Sprunggelenk in Richtung des Fußrückens zu bewegen; und
(ii) Aufblasbare und nicht-aufblasbare Abschnitte, wobei die aufblasbaren Abschnitte ausgelegt sind, um Drücken von bis zu einer Atmosphäre ohne Gasleckage oder Platzen zu widerstehen.

12. Aufblasbare Schiene (20, 220) gemäß einem der vorstehenden Ansprüche, umfassend aufblasbare und nicht-aufblasbare Abschnitte, wobei die aufblasbaren Abschnitte ausgelegt sind, um Drücken von bis zu einer Atmosphäre ohne Gasleckage oder Platzen zu widerstehen, wobei zumindest die aufblasbaren Abschnitte aus mit Polyurethan beschichtetem Nylon hergestellt sind.

13. Aufblasbare Schiene (20, 220) gemäß einem der vorstehenden Ansprüche, umfassend Fußrand-Schutzplatten.

14. Aufblasbare Schiene (20, 220) gemäß einem der vorstehenden Ansprüche 6 bis 13, wobei die Stützplatte (43) zum Anheben der Ferse mindestens eine der folgenden Konfigurationen aufweist:
Entfernbar;
Aufblasbar;
Nicht-aufblasbar aus einer weichen, flexiblen Matrix; und
Fest innerhalb der Schiene (20, 220).

15. Aufblasbare Schiene (20, 220) gemäß einem der vorstehenden Ansprüche, umfassend aufblasbare und nicht-aufblasbare Abschnitte, wobei die aufblasbaren Abschnitte einen im Wesentlichen durchgehenden aufblasbaren Gürtel, einem Rand der Schiene (20, 220) benachbart, festlegen.

## Revendications

1. Attelle gonflable (20, 220) configurée pour être placée sur une partie de jambe comprenant un pied, une cheville et un mollet de sorte que des faces avant de la partie de jambe soient exposées, l'attelle (20, 220) comprenant des parties intégrales comprenant une partie de réception de pied (24), une partie de réception de mollet (22) et une partie intermédiaire de réception de cheville (26), l'attelle (20, 220) ayant un état replié et ayant un état ouvert dans lequel elle peut être montée sur la partie de jambe, l'attelle (20, 220) ouverte prenant la forme générale de la partie de jambe, l'attelle (20, 220) étant **caractérisée en ce que** ladite partie de réception de pied (24) comporte un panneau rigide d'appui au sol (40, 240) et un coussin de pied (42) disposé au-dessus dudit panneau.

2. Attelle gonflable (20, 220) selon la revendication 1, configurée afin de prendre la forme générale de la partie de jambe dans l'état gonflé et dans l'état non gonflé.

3. Attelle gonflable (20, 220) selon la revendication 1 ou 2, dans laquelle ledit coussin de pied (42) disposé au-dessus dudit panneau dans la partie de réception de pied (24) est gonflable.

4. Attelle gonflable (20, 220) selon la revendication 3, comprenant une poche définie sous le panneau de pied pour recevoir au moins un accessoire d'attelle.

5. Attelle gonflable (20, 220) selon la revendication 4, dans laquelle ledit accessoire est un élément de stabilisation à balancier (100) destiné à être attaché à l'attelle (20, 220) pour empêcher la rotation axiale de la jambe.

6. Attelle gonflable (20, 220) selon l'une quelconque des revendications précédentes, comprenant un coussinet rembourré de support surélevant le talon (43).

7. Attelle gonflable (20, 220) selon l'une quelconque des revendications précédentes, dans laquelle des parties de l'attelle (20, 220) ont des surfaces latérales (30, 32) qui en utilisation sont disposées sur des côtés de la partie de jambe avec des bords (30D, 32D) qui définissent entre eux les faces avant exposées, lesdites surfaces latérales (30, 32) étant pourvues d'une ou plusieurs premières fixations réglables qui relient des surfaces latérales opposées l'une à l'autre, une extrémité des premières fixations étant reliée à un élément d'ancrage (60) qui est raccordé dans un agencement détachable à l'une des surfaces latérales (30, 32).

8. Attelle gonflable (20, 220) selon la revendication 7, **caractérisé par** au moins l'un de ce qui suit :
(i) ledit agencement détachable est une fermeture éclair (60C) ou un agencement de fermeture à crochet ou une boucle à ouverture rapide ;
(ii) lesdites premières fixations sont disposées sur des surfaces latérales de ladite partie de réception de mollet (22) ; et
(iii) lesdites premières fixations sont disposées sur des surfaces latérales de la partie de réception de mollet (22), et une ou plusieurs deuxièmes fixations sont fournies pour relier des surfaces latérales opposées de la partie de réception de pied (24).

9. Attelle gonflable (20, 220) selon l'une quelconque des revendications 5 à 8, dans laquelle ledit élément de stabilisation à balancier (100) est configuré pour pouvoir être attaché à l'attelle (20, 220) d'une manière détachable, et est configuré pour être attaché sur une surface inférieure de la partie de réception de pied (24), l'élément de stabilisation à balancier (100) ayant un bord qui une fois raccordé est positionné à l'arrière de la partie de réception de pied (24) et fait saillie latéralement à partir de l'attelle (20, 220).

10. Attelle gonflable (20, 220) selon la revendication 9, dans laquelle l'attachement détachable s'effectue par le biais d'un agencement de fermeture à crochet, ledit élément de stabilisation à balancier (100) ayant l'une des configurations suivantes :
(a) une surface d'attachement qui s'étend longitudinalement sur une partie substantielle de ladite face inférieure ;
(b) une langue (110) s'étendant vers l'arrière à partir dudit bord et étant configurée pour s'attacher à une surface d'attachement dans une face arrière de l'attelle (20, 220) ; et
(c) ledit élément de stabilisation à balancier (100) étant pliable.

11. Attelle gonflable (20, 220) selon l'une quelconque des revendications précédentes, comprenant au moins l'une de ce qui suit :
(i) des sangles réglables (50, 52) entre la partie de réception de pied (24) et la partie de cheville pour la dorsiflexion de l'articulation de cheville ; et
(ii) des parties gonflables et non gonflables, lesdites parties gonflables étant conçues pour soutenir des pressions jusqu'à 1 atmosphère sans fuite de gaz et sans éclatement.

12. Attelle gonflable (20, 220) selon l'une quelconque des revendications précédentes, comprenant des parties gonflables et non gonflables, lesdites parties gonflables étant conçues pour soutenir des pressions jusqu'à 1 atmosphère sans fuite de gaz et sans éclatement, dans laquelle au moins lesdites parties gonflables sont constituées de nylon enduit de polyuréthane.

13. Attelle gonflable (20, 220) selon l'une quelconque des revendications précédentes, comprenant des coussinets de protection de bordure de pied.

14. Attelle gonflable (20, 220) selon l'une quelconque des revendications 6 à 13, dans laquelle le coussinet de support surélevant le talon (43) a au moins l'une des configurations suivantes :
est amovible ;
est gonflable ;
est non gonflable en étant constitué d'une matrice flexible souple ; et
est fixe à l'intérieur de l'attelle (20, 220).

15. Attelle gonflable (20, 220) selon l'une quelconque des revendications précédentes, comprenant des parties gonflables et non gonflables, lesdites parties gonflables définissant une ceinture gonflable sensiblement continue adjacente à un bord de l'attelle (20, 220).
